# EUROPEAN PATENT SPECIFICATION

(11) **EP 4 524 258 B1**
(45) Date of publication and mention of the grant of the patent: **28.10.2026**
(21) Application number: 24199861.6
(22) Date of filing: 11.09.2024
(51) Int. Cl.: C12Q 1/6806

(54) **METHOD OF PURIFYING AND ANALYZING NUCLEIC ACID**
VERFAHREN ZUR REINIGUNG UND ANALYSE VON NUKLEINSÄUREN
PROCEDE DE PURIFICATION ET D'ANALYSE D'ACIDE NUCLEIQUE

(30) Priority: 14.09.2023 KR 20230122320
(43) Date of publication of application: 19.03.2025
(73) Proprietor: Gwangju Institute of Science and Technology, Gwangju 61005 (KR); ATG Lifetech Inc., Seoul 08507 (KR)
(72) Inventor: CHOI, Yeongjae, 61005 Gwangju (KR); PARK, so hyun, 61005 Gwangju (KR); CHOI, eun jin, 61005 Gwangju (KR); KIM, Jihee, 21118 Incheon (KR); RYU, Taehoon, 10114 Gimpo-si (KR)
(74) Representative: Mewburn Ellis LLP

(56) References cited:
- WO-A1-2022/177273
- WO-A2-2005/080604
- CHOI HANSOL ET AL: "MOPSS: Toward high-fidelity oligonucleotides for clinical applications", vol. 12, no. 7, 1 July 2022 (2022-07-01), SE, XP093233118, ISSN: 2001-1326, Retrieved from the Internet <URL:https://onlinelibrary.wiley.com/doi/full-xml/10.1002/ctm2.809> DOI: 10.1002/ctm2.809
- CHOI HANSOL ET AL: "Purification of multiplex oligonucleotide libraries by synthesis and selection", NATURE BIOTECHNOLOGY, NATURE PUBLISHING GROUP US, NEW YORK, vol. 40, no. 1, 29 July 2021 (2021-07-29), pages 47 - 53, XP037667057, ISSN: 1087-0156, [retrieved on 20210729], DOI: 10.1038/S41587-021-00988-3

## Description

### [Technical Field]

The present invention relates to a method for purifying a nucleic acid and a method for analyzing the presence of a target nucleic acid in a sample, and relates to a method for purifying a target nucleic acid from a nucleic acid library to high purity or a method for analyzing the presence of a target nucleic acid in a sample. Specifically, the present invention relates to a method for purifying and analyzing a nucleic acid for selecting a target nucleic acid based on whether a nucleic acid complementary to the target nucleic acid is synthesized.

### [Related Art]

Nucleic acids are becoming increasingly important. DNA or RNA has nucleic acids as carriers of genetic information, and the discovery of microRNAs, long noncoding RNAs, and chemically modified RNAs, for example, has reshaped our understanding of the importance of RNAs in biological processes and disease, and has led to a growing number of medical uses of RNAs (Cell, 169 (2017), pp. 1187-1200).

Most studies can be determined by high-quality nucleic acids that are free of contaminants. For this reason, research on purification continues. Purification of all or partial nucleic acids, e.g., DNA, RNA (e.g., mRNA, small RNA), or synthetic RNA isolated from a biological sample is typically performed by gel electrophoresis (RNA, 10 (2004), pp. 889-893), liquid chromatography (J. Chromatogr. A, 1216 (2009), pp. 1377-1382), organic extraction (Cold Spring Harb. Protoc, 2010 (2010)), or purification via spin column (PLoS One, 13 (2018), Article e0203011).

In particular, purified nucleic acid sequences are needed to study nucleic acid modifications within specific sequences or to study biomolecular interactions of nucleic acids by single-molecule methods.

Nevertheless, the purification rate of nucleic acids, especially RNA, by current universal purification methods is less than 80%. In particular, purification of RNA with one-base error is currently not possible.

To make RNA useful without side effects, research is needed to separate normal RNA from RNA with one-base error that occurs during synthesis, and to purify or analyze it.

Under this technical background, the inventors of the present invention have developed a technique to identify target RNAs, specifically RNAs with one-base error, and have completed the present invention.

Choi et al., Nature Biotechnology (vol. 40, no. 1, 29 July 2021, pp. 47-53) discloses purification of multiplex oligonucleotide libraries by synthesis and selection.

WO 2022/177273 A1 discloses a method for purifying a nucleic acid library.

WO 2005/080604 A2 discloses genetic analysis by sequence-specific sorting.

### [Detailed Description of the Invention]

### [Technical Problem]

It is an object of the present invention to provide a method for purifying a target nucleic acid from a library comprising the target nucleic acid.

It is an object of the present invention to provide a method for analyzing the presence of a target nucleic acid in a sample.

### [Technical Solution]

To accomplish the above objectives, the present invention provides methods recited in claims 1 to 11.

Accordingly, the present invention provides a method for purifying a target nucleic acid, comprising:
(a) hybridizing a library containing the target nucleic acid with a primer that binds complementarily to the target nucleic acid;
(b) reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid, wherein, when one of dATP, dCTP, dGTP and dTTP is selected as a blocker-free dNTP that binds complementarily to a predetermined nucleotide of the target nucleic acid, the NTPs other than the selected blocker-free NTP comprise an irreversible blocker,
   wherein the elongation reaction comprises alternating reaction cycles of NTPs comprising an irreversible blocker and blocker-free NTPs; and
(c) isolating the target nucleic acid to which the complementary nucleic acid is bound from the reactants of step (b), wherein the NTPs comprising the irreversible blocker of step (b) are dideoxy nucleoside triphosphates (ddNTPs) or pyrrolidinyl nucleoside triphosphates (prNTPs).

The present invention also provides a method for analyzing the presence of a target nucleic acid in a sample, comprising:
(a) hybridizing a sample containing the target nucleic acid with a primer that binds complementarily to the target nucleic acid;
(b) reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid, wherein, when one of dATP, dCTP, dGTP and dTTP is selected as a blocker-free NTP that binds complementarily to a predetermined nucleotide of the target nucleic acid, the NTPs comprising an irreversible blocker have other than the selected blocker-free NTP,
   wherein the elongation reaction comprises alternating reaction cycles of NTPs comprising an irreversible blocker and blocker-free NTPs; and
(c) isolating the target nucleic acid to which the complementary nucleic acid is bound from the reactants of step (b),
wherein the NTPs comprising the irreversible blocker of step (b) are dideoxy nucleoside triphosphates (ddNTPs) or pyrrolidinyl nucleoside triphosphates (prNTPs).

### [Advantageous Effects]

According to the present invention, a method for purifying or analyzing biologically useful nucleic acids to high purity can be provided.

### [Brief Description of Drawing]

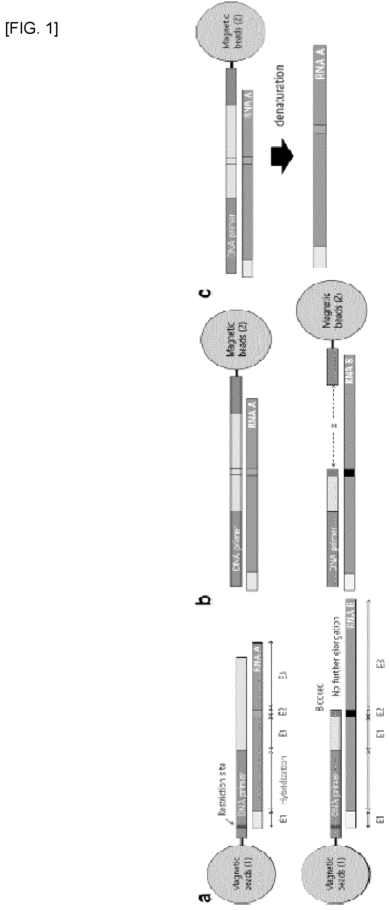

FIG. 1 shows the detailed process of RNA MOPSS (multiplex oligonucleotide library purification by synthesis and selection):
   (a) selective elongation for RNA on beads;
   (b) tagging only unblocked DNA/RNA for selective collection onto another bead;
   (c) RNA collected as follows:
      a. chemical reactions (Hybridization, Elongation/Blocking, Restriction) and conditions for MOPSS on magnetic beads proceed without damage to RNA (FIG. 1a).
      b. collecting the separated RNA by connecting it with another magnetic bead (FIG. 1b)
      c. collected RNA (FIG. 1c).
FIG. 2 shows the results of optimizing efficiency by introducing Azido-dNTP.
FIG. 2a shows the hybridization product for the introduction of Azido-dNTP.
FIG. 2b shows the resulting DNA primer without elongation when Azido-dNTP is introduced.
FIG. 2c shows the resulting long lengths of DNA identified in the case of low Azido-dNTP introduction efficiency.
FIG. 2d shows the incorporation yield for the solution condition.
FIG. 3 shows the results of optimizing efficiency by introducing ddNTP.
FIG. 3a shows the hybridization results for the initiation of ddNTP introduction.
FIG. 3b shows the results of the introduction of ddNTP, where the DNA primer did not extend, resulting in the identification of short lengths of DNA.
FIG. 3c shows the resulting long lengths of DNA identified in the case of low ddNTP introduction efficiency.
FIG. 4 shows the results of determining the introduction efficiency of ddATP, ddTTP, ddCTP, and ddGTP for different concentrations.
FIG. 5 shows the results of ddATP treatment, dNTP introduction, and endonuclease V treatment to confirm that only DNA corresponding to a specific RNA was selected and extended.
FIG. 6 shows the results demonstrating the effectiveness of selective purification via ligation.
FIG. 7 shows the structure simulation results of sgRNA and pegRNA at room temperature (20 °C) after MOPSS.
FIG. 8 shows the results of MOPSS on sgRNA, which confirmed that sequentially ordered dNTP can elongate.
FIG. 9 shows the results of determining whether the ddNTP can selectively bind only to the corresponding base and prevent the dNTP elongation from proceeding.
FIG. 10 shows the gel image results after MOPSS of 2 cycles, 4 cycles, 6 cycles, and 8 cycles with Biotin-dNTP attached to the end, and DNA and RNA collected with streptavidin bead after the corresponding MOPSS.
FIG. 11 shows the results of the analysis based on NGS data for the output of 8 cycles of MOPSS.
FIG. 12 shows the results showing that MOPSS is also possible for pegRNA.

### [Detailed Description of the Invention]

Unless otherwise defined, all technical and scientific terms used herein shall have the same meaning as commonly understood by those skilled in the art. In general, the nomenclature used herein is well known and in common use in the art. In general, the nomenclature used herein is well known and commonly used in the art.

The present invention relates to a method of purifying a target nucleic acid, in one aspect, comprising:
(a) hybridizing a library containing the target nucleic acid with a primer that binds complementarily to the target nucleic acid;
(b) reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid, wherein, when one of dATP, dCTP, dGTP and dTTP is selected as a blocker-free dNTP that binds complementarily to a predetermined nucleotide of the target nucleic acid, the NTPs other than the selected blocker-free NTP comprise an irreversible blocker,
   wherein the elongation reaction comprises alternating reaction cycles of NTPs comprising an irreversible blocker and blocker-free NTPs; and
(c) isolating the target nucleic acid to which the complementary nucleic acid is bound from the reactants of step (b),
wherein the blocking nucleotide is dideoxy nucleoside triphosphate (ddNTP) or pyrrolidinyl nucleoside triphosphate (prNTP).

The present invention also relates to a method for analyzing the presence of a target nucleic acid in a sample, comprising:
(a) hybridizing a sample containing the target nucleic acid with a primer that binds complementarily to the target nucleic acid;
(b) performing cycles of primer extension wherein each cycle comprises using a blocker free dNTP nucleotide that is selected to bind complementary to a predetermined nucleotide of the target sequence, said extension being combined with the use of blocking nucleotides that bind complementary to nucleotides other than the predetermined nucleotide of the target sequence; and
(c) isolating the target nucleic acid to which the complementary nucleic acid is bound from the reactants of step (b),
wherein the NTPs comprising the irreversible blocker of step (b) are dideoxy nucleoside triphosphates (ddNTPs) or pyrrolidinyl nucleoside triphosphates (prNTPs).

A "target nucleic acid" is a target aiming nucleic acid to be detected, which may include, but is not limited to, RNA or DNA, provided that the nucleic acid comprises a nucleotide as its basic building block. According to the present invention, it is possible to specifically distinguish RNA with a one-base error.

In the case of DNA, it may be synthesized from deoxyribonucleotides, and in a sample containing DNA, it may be DNA that is substantially free of errors or damage, and may be a gene for a desired function in an organism.

In the case of RNA, in a sample containing RNA that is synthesized from ribonucleotides, but in a modified form due to some of the nucleosides introduced during the synthesis process, it may be RNA that is substantially free of errors or damage, and may be a gene for a desired function in an organism. The target nucleic acid may be described interchangeably with the target aiming nucleic acid.

"Purification" may refer to the process of removing erroneous or damaged nucleic acids contained in a library other than the intended target nucleic acid to obtain the target nucleic acid.

"Analysis" may refer to the process of removing erroneous or damaged nucleic acids other than the target nucleic acid from a sample to determine the presence of the target nucleic acid.

A substantially error- or damage-free nucleic acid is a nucleic acid that does not contain errors, which may mean an original nucleic acid having the intended sequence. The target nucleic acid is a substantially error- or damage-free nucleic acid selected from a library comprising the target nucleic acid, which may be a nucleic acid isolated from a nucleic acid containing an error in the library, such as a nucleic acid containing an one-base error.

The library means a set of nucleic acid products and refers to nucleic acids present in the original sample. The library may comprise a nucleic acid comprising an error and a target nucleic acid, and only the target nucleic acid may be selected from the library.

"DNA" means a polynucleotide comprising a deoxyribose sugar and consisting of a purine or pyrimidine base of adenine, thymine, cytosine, or guanine.

"RNA" refers to a polynucleotide comprising a ribose sugar, and usually uracil rather than thymine as one of the pyrimidine bases. RNA can include a single-stranded molecule that is transcribed from DNA and have a linear sequence of nucleotide bases complementary to the transcribed DNA strand.

Specifically, the nucleic acid may comprise a guide RNA. It can contain a single and/or double guide RNA sequence. "dgRNA" refers to a dual guide RNA sequence comprising crRNA (a spacer sequence comprising a seed region complementary to the target sequence) and a separate tracrRNA (transcriptional activation sequence) complementary to the RNA; and "sgRNA" refers to a single guide RNA sequence containing the fused crRNA and tracrRNA.

Specifically, the nucleic acid may comprise pegRNA. pegRNA has an extension arm attached to a traditional guide RNA. In this case, the extension arm includes the extension area. The extension area includes an editing template that is a template for the intended editing, for inserting the intended editing into the target region."Nucleotide" means a glycoside comprising a sugar moiety, a base moiety, and a covalently linked linker, such as a linker between phosphate or phosphorothioate nucleotides. It can include both naturally occurring nucleotides, such as DNA or RNA, and non-naturally occurring nucleotides, which in some cases include modified sugar and/or base moieties.

The term "ribonucleotide" or "deoxyribonucleotide" can include both natural and synthetic, unmodified and modified ribonucleotides or deoxyribonucleotides. Modifications can include changes to sugar moieties, base moieties, and/or linkages between ribonucleotides or deoxyribonucleotides within an oligonucleotide.

"Polynucleotide" means a nucleotide in polymeric form that includes nucleotides of any length, including deoxyribonucleotides and/or ribonucleotides, or analogs thereof. Polynucleotide can have any three-dimensional structure and can perform any known or unknown function. The structure of polynucleotide can be considered with reference to its 5' or 3' end, which indicates the orientation of the polynucleotide. In a strand of polynucleotide, neighboring nucleotides are typically linked by a phosphodiester bond between the 3' and 5' carbons. However, other intra-nucleotide bonds can also be used, such as methylene, phosphoramidate bonds, and the like. This means that each of the 5' and 3' carbons can be exposed at both ends of the polynucleotide, which can be referred to as the 5' and 3' ends or termini. The 5' and 3' ends can also be referred to as phosphoryl (PO₄) and hydroxyl (OH) ends, respectively, due to the chemical groups attached to those ends.

The polynucleotide may comprise both double-stranded and single-stranded molecules. Examples of polynucleotides include, but are not limited to: a gene or gene fragment (e.g., a probe, primer, EST, or SAGE tag), genomic DNA, genomic DNA fragment, exon, intron, messenger RNA (mRNA), transfer RNA, ribosomal RNA, ribozyme, cDNA, recombinant polynucleotide, branched polynucleotide, plasmid, vector, isolated DNA of any sequence, isolated RNA of any sequence, nucleic acid probe, primer, or amplified copy of any of the foregoing. Polynucleotides can include modified nucleotides, such as methylated nucleotides and nucleotide analogs (including nucleotides with non-natural bases, nucleotides with modified natural bases, such as aza- or deza-purines, and the like).

The polynucleotide may be further modified after polymerization by conjugation with a labeling component. The nucleotide sequence of polynucleotide can be interrupted by non-nucleotide components. One or more ends of polynucleotide can interact in a specific way with other polynucleotides (e.g: covalent bond formation) or otherwise modified.

The polynucleotide may comprise a specific sequence of four nucleotide bases: adenine (A), cytosine (C), guanine (G), and thymine (T). Uracil (U) can exist as a natural substitute for thymine, for example, when the polynucleotide is RNA. Uracil can also be used in DNA. Therefore, the term "sequence" refers to the alphabetical representation of a polynucleotide or any nucleic acid molecule, including natural and non-natural bases.

"Primer" means a naturally occurring or synthetically produced single-stranded oligonucleotide that, when placed under conditions that catalyze the synthesis of a nucleic acid strand and a complementary primer extension product or amplicon, can serve as a synthetic initiation point along the complementary strand.

These conditions can include the presence of four different deoxyribonucleoside triphosphates and a polymerization inducer, such as thermostable nucleotide polymerase, DNA polymerase, or reverse transcriptase, in an appropriate buffer and at an appropriate temperature. Primer is preferably single-stranded to maximize amplification efficiency.

The primer binds complementarily to the target RNA. "Complementary" means sufficiently complementary that the primers selectively hybridizes to the target sequence under the prescribed annealing conditions, and includes both substantially complementary and perfectly complementary.

The term "substantially complementary sequence" as used in connection with the primer includes not only fully matched sequences, but also sequences that are partially mismatched to the sequence to be compared, to the extent that they can be annealed to a specific sequence to serve as primers.

"Annealing" or "priming" means the apposition of an oligodeoxynucleotide or nucleic acid to a template nucleic acid, wherein the apposition causes polymerization of the nucleotide to form a nucleic acid molecule complementary to the template nucleic acid or a portion thereof.

The primer should be long enough to prime the synthesis of the extension product. The appropriate length of a primer is determined by a number of factors, such as temperature, application, and the source of the primer, but is typically 15-30 nucleotides. Short primer molecules generally require lower temperatures to form sufficiently stable hybridization complexes with the template.

The design of the primer is facilitated by reference to the nucleotide sequence and can be performed, for example, using commercially available primer design programs.

"Hybridization" refers to the process by which complementary nucleotide sequences anneal to each other; specifically, the single strand of a nucleic acid sequence forms a double-helix fraction through hydrogen bonding between complementary bases.

**It** refers to the formation of double-stranded nucleic acids from complementary single-stranded nucleic acids. Hybridization can occur when there is a perfect match of complementarity between the two nucleic acid strands, or it can occur even when some mismatched bases are present.

The degree of complementarity required for hybridization can vary depending on the hybridization conditions, which can be controlled by temperature, in particular.

In a hybridization reaction, the conditions used to achieve a strict specific level vary depending on the nature of the hybridized nucleic acids. For example, the length of the nucleic acid sites to be hybridized, the degree of homology, the nucleotide sequence composition (e.g., GC/AT ratio), and the type of nucleic acid (e.g., RNA, DNA) are considered in selecting hybridization conditions.

Examples of very stringent conditions include 2X SSC/0.1% SDS at room temperature (hybridization condition); 0.2X SSC/0.1% SDS at room temperature (low stringency condition); 0.2X SSC/0.1% SDS at 42°C (moderate stringency condition); 0.1X SSC at 68°C (high stringency condition). The washing process may be performed using any one of these conditions, e.g., high stringency condition, or each of the above conditions, and these conditions in whole or in part may be repeated in the order described above for 10 to 15 minutes each. However, as described above, optimal conditions vary depending on the particular hybridization reaction involved therein and can be determined by experimentation.

"NTP" refers to nucleoside triphosphates, including not only natural (deoxy) triphosphates but also NTPs that can have additional modifications to the triphosphate side chain (e.g., methyl phosphonate, phosphothioate). It can also include a modified sugar or sugar analog (e.g., a 2'-O-alkyl derivative 3' and/or 5' aminoribose, locked ribose, hexitol, altitol, cyclohexene, cyclopentane) or a modified base, for example, 5-methyl-C or a base analog such as 7-deazapurine. The base, sugar, or triphosphate side chains of these NTPs can be linked via linkers to detectable units or reactive groups. Suitable NTPs can include both naturally occurring and synthetic nucleotide triphosphates, and can be, for example, ATP, CTP, GTP, TTP, or ITP, but are not limited thereto.

An "oligonucleotide" may include not only (deoxy)oligoribonucleotide but also oligonucleotide analogs that contain one or more nucleotide analogs modified in the phosphate backbone (e.g., methylphosphonates, phosphorothioates), or modified bases such as 7-deazapurines (e.g., 2'-O-alkyl derivatives, 3' and/or 5' amino ribose, LNA, HNA, TCA), or sugars (e.g., 2'-O-alkyl derivatives, 3' and/or 5' amino ribose).

"Nucleoside" means a compound consisting of purine, deazapurine, or pyrimidine nucleoside base, for example, adenine, guanine, cytosine, uracil, thymine, deazadenine, deazaguanosine, or the like, linked to pentose at the 1' position, including the 2'-deoxy and 2'-hydroxyl forms.

With respect to adenine, guanine, cytosine, uracil, and thymine, the symbols listed are as follows:
A - Adenine;
C - Cytosine;
DNA - Deoxyribonucleic acid;
G - Guanine;
RNA - Ribonucleic acid;
T - Thymine; and
U - Uracil.

"Preparation" is an object to be reacted with a hybridization reactant and may include, but is not limited to, any substance that can be a component of the reaction. The preparation comprises a nucleotide triphosphate (NTP) comprising an irreversible blocker.

Step (b) comprises sequentially alternating reaction cycles of NTPs comprising an irreversible blocker and blocker-free dNTPs.

With respect to any one of the blocker-free dNTPs selected from the group consisting of blocker-free dATP, dCTP, dGTP, and dTTP that binds complementarily to a predetermined nucleotide of the target nucleic acid, the blocker-free dNTP is an unmodified dNTP. The blocker-free dNTP is one of four deoxyribonucleotide triphosphates: dATP (deoxyadenosine triphosphate), dCTP (deoxycytidine triphosphate), dGTP (deoxyguanosine triphosphate), and dTTP (deoxythymidine triphosphate). The blocker-free dNTPs can be used as building blocks in DNA synthesis in general.

The NTP comprising the irreversible blocker is a nucleotide analog capable of terminating DNA synthesis. NTPs comprising the irreversible blocker is dideoxy nucleoside triphosphate (ddNTP) or pyrrolidinyl nucleoside triphosphate (prNTP).

The ddNTP is dideoxynucleotide, which may include, for example, ddATP, ddCTP, ddGTP, or ddTTP.

The prNTP (pyrrolidinyl nucleoside triphosphate) is a derivative of nucleoside triphosphate, wherein the nucleoside triphosphate is substituted with a 4-6 membered cycloheteroalkyl, for example, azetidinyl, pyrrolidinyl, piperidinyl, morpholinyl, thiamorpholinyl, imidazolidinyl, tetrahydrofuranyl, tetrahydropyranyl, tetrahydrothiophenyl, or piperazinyl. The prNTP may be a derivative in which the nucleoside triphosphate is substituted with a pyrrolidinyl group.

The prNTP may be, for example, prATP, prTTP, prGTP, or prCTP.

In the NTP comprising the irreversible blocker, the remaining NTPs except for one selected from the group consisting of the blocker-free dATP, dCTP, dGTP, and dTTP comprises an irreversible blocker.

Specifically, when one of dATP, dCTP, dGTP and dTTP is selected as a blocker-free dNTP that binds complementarily to a predetermined nucleotide of the target nucleic acid, the NTPs other than the selected blocker-free dNTP may comprise an irreversible blocker.

For example, when any one is selected from dNTPs that complementarily binds to a predetermined nucleotide of the target nucleic acid, for example, deoxyadenosine triphosphate (dATP), deoxycytidine triphosphate (dCTP), deoxyguanosine triphosphate (dGTP), and deoxythymidine triphosphate (dTTP), the NTPs other than the selected dATP, dCTP, dGTP or dTTP may have a ddNTP form.

If the preparation that complementarily binds to a predetermined target nucleic acid comprises dATP, it may comprise an NTP other than dATP, for example, ddCTP, ddGTP, or ddTTP.

The present invention provides a method for purifying a target RNA from a library comprising the target RNA, wherein the method can be applied to RNA by multiplex oligonucleotide library purification by synthesis and selection (MOPSS). The present invention includes the steps of (a) hybridizing a library comprising a target nucleic acid with a primer that binds complementarily to the target nucleic acid.

The library may comprise a nucleic acid comprising an error and a target nucleic acid. By hybridizing a primer that complementarily binds to the target nucleic acid, the primer having a sequence complementary to the target nucleic acid may bind.

The primer complementarily binds to the target nucleic acid and may be further linked with a dye to confirm binding. The dye may be ligated to the 5' end of the primer.

The dye is not limited as long as the binding to the target RNA can be identified via fluorescence or light, but includes, for example, FITC, Alexa Fluor 488, GFP, CFSE, CFDA-SE, DyLight 488, PE, PI, PerCP, PerCP-Cys 5.5, PE-Alexa Fluor 700, PE-Cy5 (TRI-COLOR), PE-Cy5. 5, PE-Alexa Fluor 750, PE-Cy7, APC, APC-Cy7, APC-eFluor 780, Alexa Fluor 700, Cys5, Draq-5, Pacific Orange, Amine Aqua, Pacific Blue, DAPI, Alexa Fluor 405, eFluor 450, eFluor 605 Nanocrystals, eFluor 625 Nanocrystals, or eFluor 650 Nanocrystals.

In specific examples according to the present invention, Cy5 may be linked to the 5' end of the primer so that the resulting product can be identified by gel electrophoresis in a separate wavelength band.

The present invention comprises the step of reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid.

In the step, the hybridization reactant generated by hybridizing a library comprising a target nucleic acid with a primer that binds complementarily to the target nucleic acid in step (a) is reacted with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid.

In step (b), in the NTP comprising the irreversible blocker, the remaining NTPs except for one selected from the group consisting of the blocker-free dATP, dCTP, dGTP, and dTTP comprises an irreversible blocker.

Step (b) may comprise alternating reaction cycles of NTPs comprising an irreversible blocker and blocker-free dNTPs.

Reacting a preparation comprising the blocker-free dNTP is an elongation reaction, wherein a blocker-free deoxy NTP selected from the group consisting of complementary blocker-free dATP, dCTP, dGTP, and dTTP, is coupled to a target nucleic acid of n bp to a desired length, thereby increasing the length of the nucleic acid.

For example, when the sequence of the target nucleic acid is 3'-TGTGGCGGCAGATGATGACGcgggctgctctgatggccaagctataccctaagctgataACCCCAGTCCCCT GCTGTCA-5', to purify the target nucleic acid, the elongation reaction can be initiated after hybridizing a portion of the target nucleic acid with a complementary sequence 5'-ACACCGCCGTCTACTACTGC-3'.

The complementary sequence 5'-ACACCGCCGTCTACTACTGC-3' hybridizes to the 3'- -5' site, and if only one of the complementary blocker-free dNTPs from among the blocker-free dNTPs, in the order of the target nucleic acid sequence, is subsequently bound, binding will not occur in molecules with a different sequence from the target nucleic acid sequence, resulting in a different length of the complementary strand.

For example, hybridization of the complementary sequence and binding of only dGTP will result in binding to the target nucleic acid sequence but not to the sequence that does not contain c, resulting in a difference in the length of the complementary sequence.

If it is desired to use the same mechanism to bind dCTP after dGTP, the target nucleic acid will bind three dCTPs, but if there is an error in ggg, less than three dCTPs will bind.

In this regard, even if using only blocker-free dNTP, if the order of blocker-free dNTP to bind is determined based on the complementary sequence of the target nucleic acid, the molecule containing the synthesis error will not bind, leading to a difference in the length of the complementary strands, and this difference in length can be used to purify (screen) the target nucleic acid.

This introduces a blocker-free dNTP that binds complementarily to a predetermined nucleotide of the nucleic acid template to which the primer has hybridized, allowing the nucleic acid to be extended to the desired length.

The nucleic acid may be synthesized on a solid support, e.g., glass, beads, particles, or other suitable forms. The material composition of the solid support can be any suitable material, such as a polymeric or silica-based support. For example, it can include plastic, nylon, glass, silica, metal, metal alloy, polyacrylamide, polyacrylate, polystyrene, cross-linked dextran, and combinations thereof.

The reaction is an amplification reaction, meaning a reaction to obtain a replication product of nucleic acid. It can be generated by any of a variety of amplification methods using the target nucleic acid as a template.

This method involves using one of the blocker-free deoxy NTPs selected from the group consisting of blocker-free dATP, dCTP, dGTP, and dTTP, to generate a nucleic acid that binds complementarily to a base at a specific position from the 5' end of the target nucleic acid in the n bp, wherein the remaining NTPs, excluding the NTP selected from the group consisting of blocker-free dATP, dCTP, dGTP, and dTTP, include an irreversible blocker, thereby allowing the elongation of the nucleic acid to be either permanently or temporarily halted at any position from 5' to 3'.

The reaction of the above step may result in the binding of a nucleic acid complementary to the target nucleic acid and mismatch to the non-target nucleic acid, such that the reaction is stopped by an NTP comprising an irreversible blocker.

NTPs comprising the irreversible blocker is dideoxy nucleoside triphosphates (ddNTPs) or pyrrolidinyl nucleoside triphosphates (prNTPs).

The ddNTP may include, for example, but is not limited to, ddATP, ddCTP, ddGTP, or ddTTP.

The prNTP (pyrrolidinyl nucleoside triphosphate) may be, for example, prATP, prTTP, prGTP, or prCTP, but are not limited thereto.

The NTP comprising the irreversible blocker can be included at a concentration of, for example, 20 to 200 µM. For various concentrations, it has been shown that the irreversible blocker, such as ddATP, ddCTP, ddGTP, or ddTTP, can be introduced with high efficiency, but ddTTP is required to have concentration of 200 µM or more.

The blocker-free dNTP is one of four deoxyribonucleotide triphosphates: dATP (deoxyadenosine triphosphate), dCTP (deoxycytidine triphosphate), dGTP (deoxyguanosine triphosphate), and dTTP (deoxythymidine triphosphate).

The preparation of step (b) may further comprise, for example, an enzyme, a buffer, and a salt.

The enzyme is a polymerase, which includes an enzyme that produces complementary copies of a nucleic acid molecule utilizing the nucleic acid as a template strand. DNA polymerase binds to the template strand and then sequentially moves down the template strand, adding nucleotides to the free hydroxyl groups at the 3' end of the growing strand of the nucleic acid. Polymerase can utilize short strands, called primers, to initiate strand growth. Some polymerases can displace strands upstream of the site where they add a base to the chain. These polymerases are called strand displacers, meaning that they have the activity of removing the complementary strand from the template strand that is read by the polymerase.

The polymerase may be, for example, but not limited to, a Taq DNA polymerase, a Tth DNA polymerase, a Pfu DNA polymerase, a Bst DNA polymerase, a Tli DNA polymerase, a KOD DNA polymerase, an nTha DNA polymerase, or a Tha DNA polymerase.

The buffer may be any buffer suitable for the reaction, and may include, but is not limited to, for example, a pH-stabilizing solution such as Tris, CAPS (N-cyclohexyl-3-aminopropanesulfonic acid), HEPES (4-(2-hydroxyethyl)-1-piperazineethanesulfonic acid), MOPS (3- morpholinopropane-1-sulfonic acid), CAPSO (3-(Cyclohexylamino)-2-hydroxy-1-propanesulfonic acid), EPPS (4-(2-Hydroxyethyl)-1-piperazinepropanesulfonic acid), MES (2-(N-Morpholino)ethanesulfonic acid), PIPES (1,4-Piperazinediethanesulfonic acid), TAPS (N-[Tris(hydroxymethyl)methyl]-3-aminopropanesulfonic acid), TAPSO (2-Hydroxy-3-[tris(hydroxymethyl)methylamino]-1-propanesulfonic acid), TES (2-[(2-Hydroxy1,1-bis(hydroxymethyl)ethyl)amino]ethanesulfonic acid), or PBS (Phosphate Buffered Saline).

The salt may comprise, for example, MnCl₂ or MnSO₄. In this case, the salt may be included at a concentration of 1 mM or more. The salt may be included at a concentration of, for example, 10 mM or less.

Because RNA has a different chemical structure than DNA, there are differences in the conditions under which reactions can occur with RNA as a template and the resulting reactivity. The introduction of MnCl₂ may not be necessary to synthesize complementary sequences to DNA. However, for complementary DNA synthesis to RNA, the introduction of MnCl₂ may be necessary when reacting a preparation containing (i) a nucleotide triphosphate (NTP) comprising an irreversible blocker an (ii) a blocker-free dNTP.

In specific examples according to the present invention, it has been found that when the concentration of MnCl₂ in the preparation is greater than or equal to 1 mM, the incorporation yield of NTP comprising irreversible blocker and/or cleavable blocker can approach 100%.

Step (b) is not limited as long as the conditions are sufficient to allow the reaction of the hybridization reactants of step (a) with the preparation to occur, but may be, for example, at a temperature of 60°C for 15 minutes.

The present invention includes the step (c) of isolating the target nucleic acid to which the complementary nucleic acid is bound from the reactants of step (b).

The elongation reaction of step (b) can be used to isolate the target nucleic acid to which the complementary nucleic acid is bound, excluding products that are mismatched and in which the reaction is stopped by the introduction of an irreversible blocker.

To perform the step (c), the target nucleic acid to which the complementary nucleic acid is bound may be separated from the product in which the reaction has stopped, including the step of ligating the additional tag.

The tag is a capture tag for isolating a target nucleic acid, and it may be intented to separate a target nucleic acid to which a complementary nucleic acid is bound from a product to which the reaction has stopped due to the introduction of an irreversible blocker that has not reacted. The tag can be biotinylated, for example, by contacting a biotinylated dideoxynucleotide to an NTP comprising an irreversible blocker that is non-complementary to the target nucleic acid to form a biotin bond only to the target nucleic acid, and selectively extracting only the target nucleic acid via particles that form a bond with biotin under appropriate binding conditions.

Specifically, biotin-dATP can be introduced using a terminal transferase used for nucleotide triphosphate (NTP) tailing that contains an irreversible blocker, and screened using streptavidin-coated magnetic particles with oligonucleotides that can form a bond with it.

The magnetic particles are available from commercial sources. The magnetic particle is a particle that is not magnetic but responds to a magnetic field by forming a magnetic dipole when exposed to a magnetic field. The magnetic particles include, for example, materials that can be magnetized in the magnetic field but are not themselves magnetic in the absence of a magnetic field. The magnetic particles may be, for example, magnetic beads. The magnetic material includes, but is not limited to, any paramagnetic or superparamagnetic material.

The method may further comprise the step (d) of isolating only the target nucleic acid from the nucleic acid to which the complementary nucleic acid is bound.

The target nucleic acid to which the complementary nucleic acid is bound may be treated with an enzyme to isolate only the target nucleic acid from the target nucleic acid to which the complementary nucleic acid is bound.

The present invention will now be described in more detail with reference to the following examples. These examples are for the purpose of illustrating the invention only, and it will be apparent to one of ordinary skill in the art that the scope of the invention is not to be construed as limited by these examples.

### Example 1: RNA MOPSS (multiplex oligonucleotide library purification by synthesis and selection)

### 1-1. RNA MOPSS process

Despite the growing medical use of RNA materials, the purification rate of RNA by universal purification methods is 80% or less. In particular, purification of RNA with one-base error is currently not possible. In order to use RNA medicinally without side effects, research is needed to separate and purify normal RNA from RNA with one-base error that occurs during synthesis.

Therefore, a new chemistry is provided along with a technique to distinguish one-base error. RNA has different physical and chemical properties than DNA, so new chemistry is applied to the reaction.

The detailed process of RNA MOPSS is shown in FIG. 1. According to FIG. 1, (a) selective elongation for RNA on a bead; (b) selective collection onto another bead by tagging only unblocked DNA/RNA; and (c) collection of only RNA are performed:
a. Chemical reactions (Hybridization, Elongation/Blocking, Restriction) and conditions for MOPSS on magnetic beads were shown to proceed without damage to RNA (FIG. 1a).
b. The separated RNA was collected by linking it to another magnetic bead (FIG. 1b).
c. Only RNA was collected (Fig. 1c).

The specific of MOPSS is as follows:
i. For magnetic beads, a DNA primer was linked via chemical bonds.
ii. Hybridization (H): RNA complementary to the DNA primer sequence was allowed to bind to it. For beads, the reactants were confined to be on the beads to allow for solution exchange.
iii. Elongation (E): The DNA was extended to the desired length by polymerase using individual dNTPs or combinations thereof over the hybridized RNA template.
iv. Blocking (B): Instead of dNTP at a specific base, an irreversible or reversible blocker, ddNTP or Azido dNTP, was inserted to stop or temporarily halt DNA elongation.
v. Isolation (Restriction, R): DNA and RNA synthesizing the desired sequence by elongation were separated in the case of beads and eluted in solution by breaking the bonds of specific sequences in the DNA sequence that are delimited by restriction enzyme sites.
vi. Capture (C): Eluted DNA/RNA was collected by linking it to another magnetic bead. Only DNA extended to a certain length was collected based on the length difference, so that blocked RNA remained in solution and was not collected.

### 1-2. RNA Screening with MOPSS

### (1) Sequence design

**[Table 1]**

| | Type | Base | Sequence (5' → 3') |
|---|---|---|---|
| DNA Primer | (1) | 37 nt | [Amine] TTTTT*IIIII* (SEQ ID NO: 1) |
| | | | [Cy5]TGGTCTC CTCCAGTCACCAAATATGCG (SEQ ID NO: 2) |
| RNA | A | 58nt | UACUCAACUUGAAAAGGUGGC A // CCGA // UUCGGUGUUUUU//CGCAUAUUGGUGACUGGAG (SEQ ID NO: 3) |
| | B | 68nt | UUUUUUUUUUACUCAACUUGAAAAGGUGGC U // CCGA // UUCGGUGUUUUU // CGCAUAUUGGUGACUGGAG (SEQ ID NO: 4) |

Target bases were highlighted (blue), primer binding sites were highlighted (yellow), and the sequence was zoned according to the elongation method.

The DNA primer was labeled with Cy5 dye, which allowed for a separate wavelength band from conventional nucleic acids staining when viewing the results by gel electrophoresis.

Blue highlights were introduced for base differences between A and B types, assuming errors in the RNA. The gray highlights were added to artificially introduce a difference in length. The restriction site, I (Inosine), was labeled in purple italics.

### (2) Introduction of Azido-dNTP

To optimize Azido-dNTP introduction efficiency, RNA type A was hybridized onto beads with DNA primer and introduction was performed under various conditions.

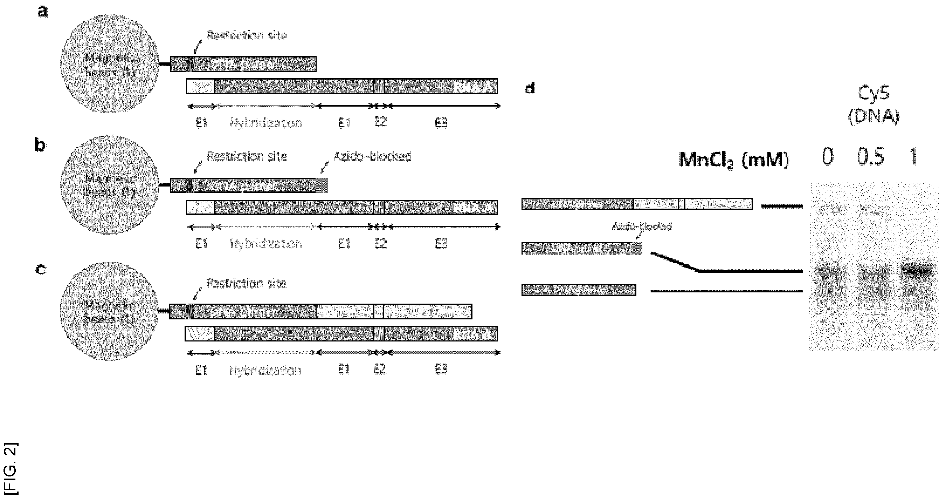

The dNTP was extended after introduction of one nucleotide (FIG. 2a) and the resulting product was subjected to restriction enzyme (endonuclease V) treatment.

If Azido-dNTPs are introduced, short DNA lengths should be seen as the DNA primer does not extend (FIG. 2b), and if the introduction efficiency is low, long lengths of DNA should be seen as in FIG. 2c.

Using 9°N DNA polymerase, it was found that Azido-dNTP was introduced under various conditions, but the efficiency was lower than 100%. The incorporation yield was found to be close to 100% when the concentration of MnCl₂ in solution was 1 mM or more (Fig. 2d). In this case, the extension condition was 15 minutes and the temperature was 60°C.

### (3) Introduction of ddNTP

**[Table 2]**

| | Type | Base | Sequence (5' → 3') |
|---|---|---|---|
| DNA Primer | (1) | 37 nt | [Amine] TTTTT*IIIII* (SEQ ID NO: 1) |
| | | | [Cy5]TGGTCTC CTCCAGTCACCAAATATGCG (SEQ ID NO: 2) |
| RNA | A | 58 nt | UACUCAACUUGAAAAGGUGGCACCGA UUCGGUGUUUUU//CGCAUAUUGGUGACUGGAG (SEQ ID NO: 5) |

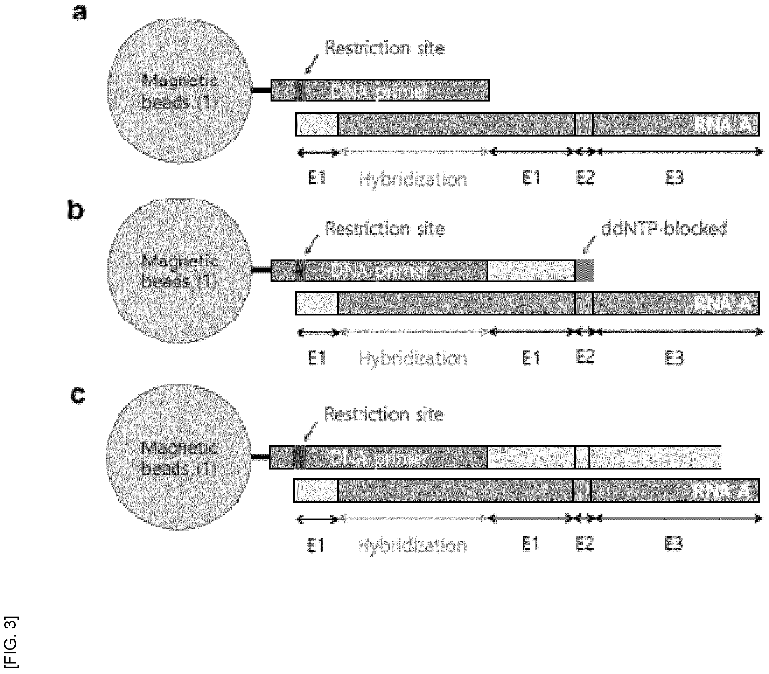

To optimize the introduction efficiency of ddNTP, RNA type A was hybridized onto beads with DNA primer, on which dATP, dCTP, dGTP, dTTP, and azido dNTPs were used to move to specific sites (highlighted green in the table above) for ddNTP introduction (FIG. 3a).

After introduction of the ddNTP, the dNTP was elongated and the resulting product was subjected to restriction enzyme (endonuclease V) treatment. If ddNTP is introduced, short DNA lengths should be seen as the DNA primer does not extend (FIG. 3b), and if the introduction efficiency is low, long lengths of DNA should be seen as in FIG. 2c (FIG. 3c).

Bst 3.0 is a polymerase known to be capable of ddNTP or dNTP introduction on both RNA and DNA Polymerase was used, and MnCl₂ was added to a final concentration of 1 mM.

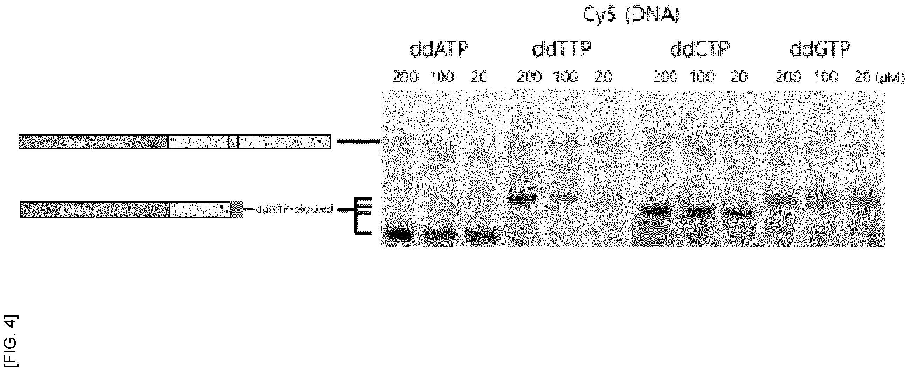

For various concentrations, it has been shown that ddATP, ddTTP, ddCTP, and ddGTP were introduced with high efficiency, but ddTTP was required to have a concentration of 200 µM or more (FIG. 4).

### Example 2: RNA screening

Based on the conditions of Examples 1-2, an experiment to select specific RNAs among type A and type B RNAs was conducted.

Type A and B RNAs can be screened by blocking them with ddNTPs because they differ in specific bases. The two types of RNA were hybridized simultaneously on beads and introduction was performed under various conditions.

To screen the positions, the first region "UUCGGGUGUUUUUU" shown in Table 2 was extended with d(C,G,A)NTP, and the subsequent region "CCGA" was extended with azido-dNTP for 4 cycles. Blocking with Azido-dNTP was removed by treatment with 100 mM of Tris(2-Carboxyethyl)phosphine (TCEP) at pH9.0 at 65°C for 15 min.

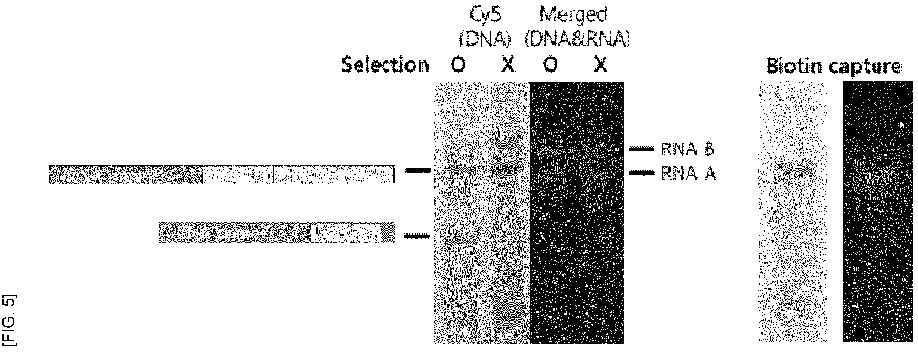

Subsequent ddATP treatment, dNTP introduction, and restriction enzyme (endonuclease V) treatment confirmed that only DNA corresponding to the specific RNA was selected and extended (FIG. 5).

Terminal transferase, which is used for DNA tailing, was then used to introduce biotin-dATP, resulting in enrichment of only RNA A using streptavidin beads.

### Example 3. Effect of selective purification via ligation

The sequence of the target RNA, the sequence of the primer for MOPSS (primer 1 below), and the sequence of the oligo used for ligation (primer 2) are shown below.
RNA : 
CUAGUUCCAGACACCUCCCAAGCACGCAGCAAUGCAGCUCAAAACGCUUAGCCU
AGCCACACCCCCCCACGGGAAACAGCAGUGAUUAACCUUUAGCAAUAA (SEQ ID NO: 6)
Primer 1 (P1, 42 mer):
   5'-biotin-TTTTTTTTTTGGTCTCTTTTTTTTTTATTGCTAAAGGTTAATCA (SEQ ID NO: 7)
Primer 2 (P2, 54 mer):
   AGGCTAAGCGTTTTGAGCTGCATTGCTGCGTGCTTGGGAGGTGTCTGGAACTAG (SEQ ID NO: 8)

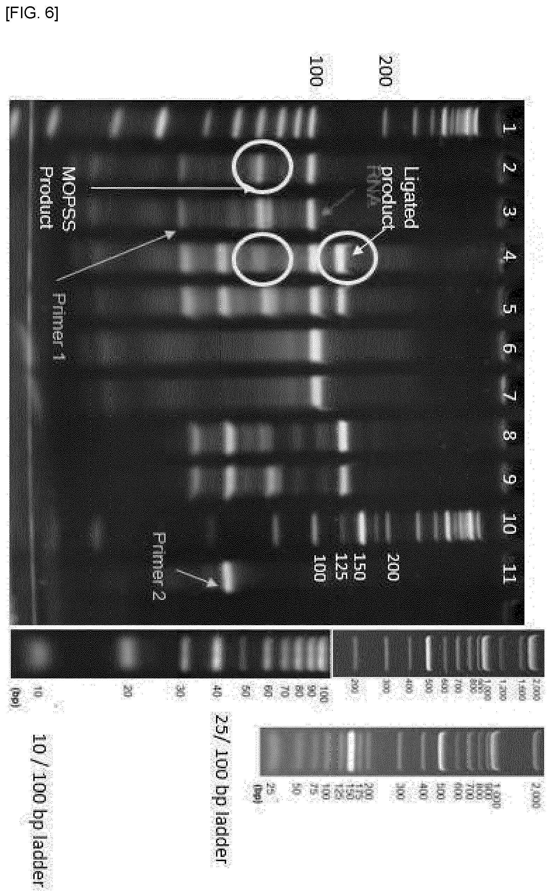

Primer 2 is phosphorylated at the 5' end for ligation, and in FIG. 6, the sample in each lane is loaded with 10 ul of primer 1, which corresponds to an amount of 10 pmol.

In addition, each sample was loaded so that the ratio of P1:RNA:P2 was 5:1:10. FIG. 6 shows the results of an experiment to determine if ligation can be used to select only error-free RNAs to select only DNA that has been elongated to a specific position after MOPSS.

In FIG. 6, the BIONEER 10/100 bp ladder is shown on the left and the 25/100 bp ladder on the right.

Table 3 shows the results of how the samples in each lane reacted.

**[Table 3]**

| Label | 1 | 2 | 3 | 4 | 5 | 6 | 7 | 8 | 9 | 10 | 11 |
|---|---|---|---|---|---|---|---|---|---|---|---|
| MOPSS Cycle | | dCTP+dTTP+dGTP+dCTP+dTTP+dGTP+dTTP+dCTP+dGTP+dTTP+dGTP+dTTP+dGTP+dTTP+dGTP+dCTP+dTTP | | | | | | | | | |
| HEAT right after MOPSS | | X | 65C 5min | X | 65C 5min | X | 65C 5min | X | 65C 5min | | |
| Ligation | | X | X | O | O | O | O | O | O | Bioneer 25/100bp ladder | |
| Primer 2 (P2) for Ligation | Bioneer 10/100bp ladder | X | X | O | | | | | | | Primer 2 for Ligation |
| Dnase I Treatment | | X | X | X | X | O | O | X | X | | |
| Rnase H&I Treatment | | X | X | X | X | X | X | O | O | | |
| Status | | Right after MOPSS (1 cycle) | | Right after Ligation | | Dnase I Treatment after Ligation | | Rnase H&I Treatment after Ligation | | | |

Lane 2 is the result of MOPSS and it can be seen that the darker band at the top and the fainter band at the bottom in the yellow circle, where the top is the DNA that has been elongated to the desired position and the bottom is the byproduct.

Lane 4 is the result of performing a ligation reaction on the sample of lane 2, and compared to the result from lane 2, it can be seen that the DNA has ligated to the desired position and lengthened (yellow circle at the top of lane 4).

It was also found that when checking the yellow circle at the bottom of lane 4, the amount of DNA that had previously extended to the desired location was significantly reduced compared to that in lane 2.

On the other hand, other products, RNA, etc. did not change in length and quantity, indicating that ligation did not occur.

Overall, from the results in FIG. 6, it can be concluded that only DNA that extended to the desired position was selectively ligated.

The results from lane 4 also confirmed that there was no length change in other products or RNA after the ligation reaction.

Lanes 6 and 8 demonstrated by treating DNase and RNase, respectively, that the product in the yellow circle above lane 4 is DNA that extends to the desired location and that the other products are RNA, primers, or DNA that does not extend to the desired location.

### Example 4. Purification of therapeutic RNAs with MOPSS

Using the CRISPR (clustered regularly interspaced short palindromic repeats) system, therapeutic RNAs that can correct DNA errors at the gene level were purified through MOPSS and the purification effect was confirmed (using the actual RNA sequence).

The therapeutic RNAs purified by MOPSS are single-guide RNAs (sgRNAs) and prime editing guide RNAs (pegRNAs).

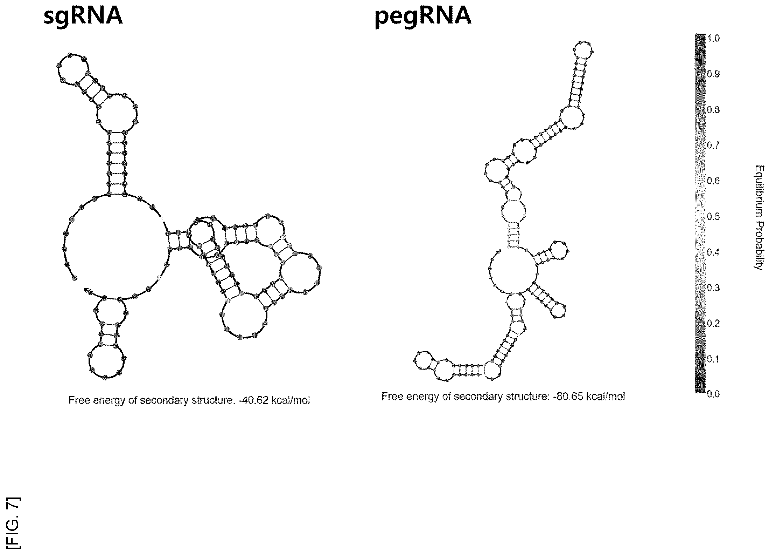

The structure simulation results of sgRNA and pegRNA at room temperature (20 °C) using MOPSS are shown in FIG. 7. It is a single strand with a length of 100 nt or more and a high GC content, but it has a complex conformation due to complementary sequences and the flexibility of the structure.

**[Table 4]**

| | Type | Base | Sequence (5' → 3') |
|---|---|---|---|
| RNA | sg | 120nt | UCC ACC AGU UUU AGA GCU AGA AAU AGC AAG UUA AAA UAA GGC UAG UCC GUU AUC AAC UUG AAA AAG UGG CAC CGA GUC GGU GCU UUU AGA UCG GAA GAG CAC ACG UCU GAA CUC CAG UCA (SEQ ID NO: 9) |
| | peg | 162nt | GGU GGA AGC UGU GUU GCC AAG UUU CAG AGC UAU GCU GGA AAC AGC AUA GCA AGU UGA AAU AAG GCU AGU CCG UUA UCA ACU UGA AAA AGU |
| | | | GGC ACC GAG UCG GUG CAG UCU GUC CAG UCA UUU GUC CAU UGG CAA CAC GGG CAG GGC UGG GAG GGU UUU UUU (SEQ ID NO: 10) |
| DNA primer | sg | 40nt | [NH2-C12]TTTTT***IIIII***[CY5]TGGTCTC TGA CTG GAG TTC AGA CGT GTG CT (SEQ ID NO: 11) |
| | peg | 49nt | [NH2-C12]TTTTTTTTTT//////[CY5] TTTAAA AAA ACC CTC CCA GCC CTG CCC GTG TTG C (SEQ ID NO: 12) |

| | | | |
|---|---|---|---|
| - Blue: Sequences that bind via hybridization between DNA and RNA. - Orange: Areas of MOPSS | | | |

For purification using MOPSS for the corresponding RNA, DNA primer binding, hybridization, blocking, elongation, restriction, and capture on the six magnetic beads described above were performed in a stepwise manner.

DNA primer complementary to a portion of the sequence in the 3' direction of the RNA to undergo MOPSS was chemically bound to the magnetic bead. The DNA primer on the bead and the corresponding RNA were combined via hybridization, immobilizing the RNA on the bead. Afterward, ddNTP blocking and dNTP elongation were conducted in an alternating manner, and MOPSS was performed sequentially according to the type of base corresponding to the sgRNA sequence.

Blocking was performed with a buffer containing 1 mM Mn2+ and a concentration of 40 uM ddNTP. Elongation was performed without Mn2+ and at a concentration of 20 uM dNTP.

The order is ddNTP, then dNTP. The ddNTP proceeds with a combination of three ddNTPs that do not contain the sequence of the subsequent dNTP. The dNTP proceeded one at a time through the known sequences.

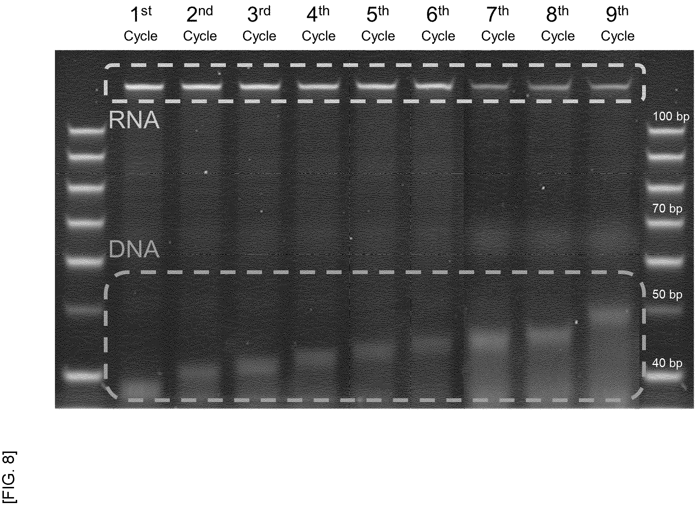

One MOPSS cycle is a set of blocking and dNTP elongation using a combination of three different ddNTPs corresponding to one base on the RNA sequence. It was confirmed that dNTPs can elongate sequentially in sgRNA (FIG. 8).

In FIG. 8, the DNA primer is labeled with Cy5, which is colored red on the gel image. RNA is labeled yellow on the gel by imaging with SYBR gold staining.

The gel shows the sequential lengthening of DNA through dNTP elongation up to cycle 9th.

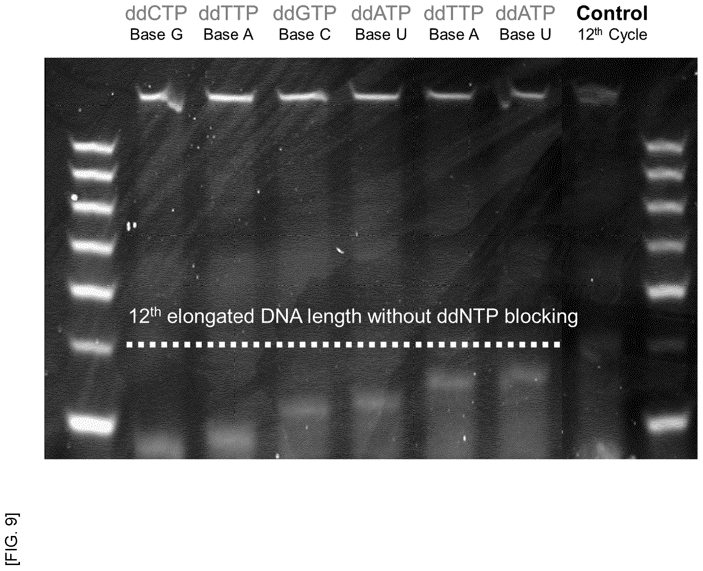

To proceed with the blocking, it was verified that the ddNTP selectively binds only to the corresponding base, preventing the progression of dNTP elongation (FIG. 9).

It was shown that blocking works perfectly for all four types of bases A, G, C, and U in the sgRNA.

Without blocking, a red DNA band that progressed to the 12^{th} cycle, which is the control (the DNA band increased to the white line in FIG. 9) would have been observed. However, a complete stop was observed at the length where the base is located. This demonstrates that the blocking is fully functional and confirms that the sgRNA is unable to elongate to the final length of DNA due to the blocking of other randomly generated bases as the MOPSS progresses.

Finally, the DNA that has undergone MOPSS to reach the target position is finally linked with biotin-dNTP at the 3' end of the DNA so that it can be selectively extracted by streptavidin magnetic beads during the capture step.

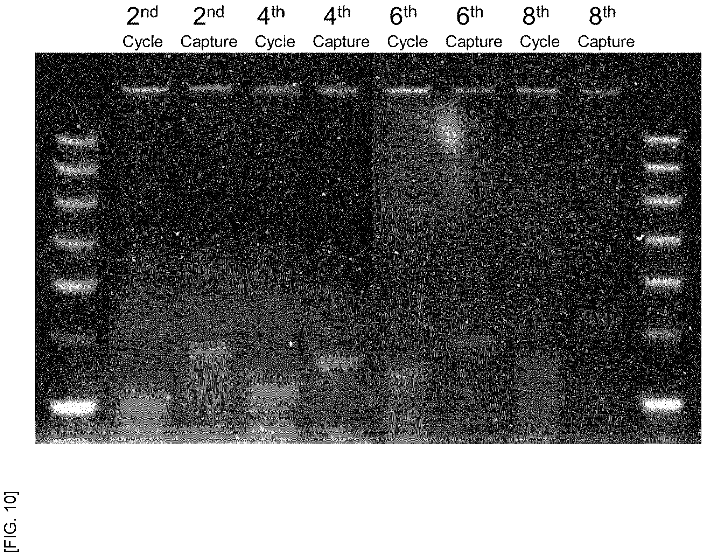

Below are gel images of DNA and RNA collected on streptavidin beads after MOPSS of 2, 4, 6, and 8 cycles with biotin-dNTPs attached to the ends (FIG. 10).

The results of the captured DNA and RNA are shown as a set for cycles 2, 4, 6, and 8, in which biotin-dNTPs are attached after each MOPSS cycle.

The results of the MOPSS cycle and collection of only DNA bands bound by biotin-dNTPs show that the pure MOPSS product can be separated and extracted by the second streptavidin magnetic bead.

To analyze the improvement of MOPSS, Next-Generation Sequencing (NGS) on RNA was performed before and after MOPSS.

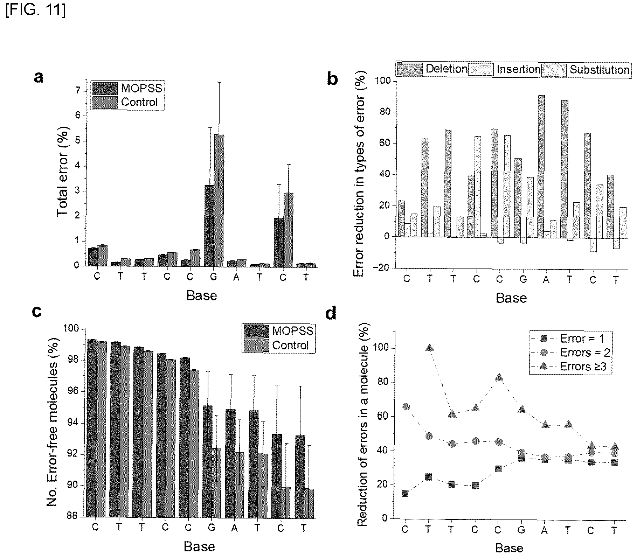

The effect of decreasing the error in bases on the sgRNA after 8 cycles of MOPSS is shown in FIG. 11 (FIG. 11a).

Results from NGS on raw RNA before MOPSS are labeled as control. The results of MOPSS are labeled as MOPSS.

The graphs in FIG. 11 show the results of an analysis based on NGS data of the results of 8 cycles of MOPSS (top graph: analysis based on the probability of a base error at each position).

The results show that MOPSS results in an overall reduction in total errors per base compared to the control (FIG. 11a). Control results show that the error rate per base is not related to length or base type. On average, MOPSS showed an error rate reduction of about 30% compared to control, regardless of these erratic error rates.

When analyzed by the types of errors that make up the total error rate (insertion, deletion, and substitution), the overall effect was to reduce the error rate (FIG. 11b). While the improvement rate varies due to the different distribution of each type of error, the overall reduction in all types of errors after MOPSS confirms the effectiveness of the purification (bottom graph: analysis on the error reduction at the molecular level). Before and after MOPSS, the increase in purity was analyzed and plotted by analyzing the percentage of molecules that did not contain errors in the analyzed sample (FIG. 11c). Compared to rawRNA (control), the percentage of pure RNA that does not contain errors increases after MOPSS.

To analyze the effect of progressing through MOPSS, the number of windows was increased one by one (e.g., C → CT → CTT → ...) and the data was analyzed accordingly. As the window increases, the number of error-containing bases increases, showing a gradual increase in the number of error-containing molecules and a corresponding decrease in the number
of error-free molecules.

It was analyzed whether the number of errors within molecules that contain errors is also reduced, as a smaller percentage of errors increase the likelihood that they will not cause significant errors in the final function (Fig. 11d).

As the window increased, the number of errors also tended to increase, and within the 8 cycles of MOPSS, the error improvement was seen by confirming how many the errors in that range decreased relative to the control as the window increased. The improvement was seen across all regions regardless of the number of errors, and when using the entire range up to 8th cycle as the window, an improvement effect of approximately 40% was observed in all error counts.

To analyze the error reduction effect mentioned above, the error reduction was calculated and plotted compared to the control using the formula (Error_{MOPSS,type} - Error_{Control,type})/ Error_{Control,type} (Fig. 11b and Fig. 11d).

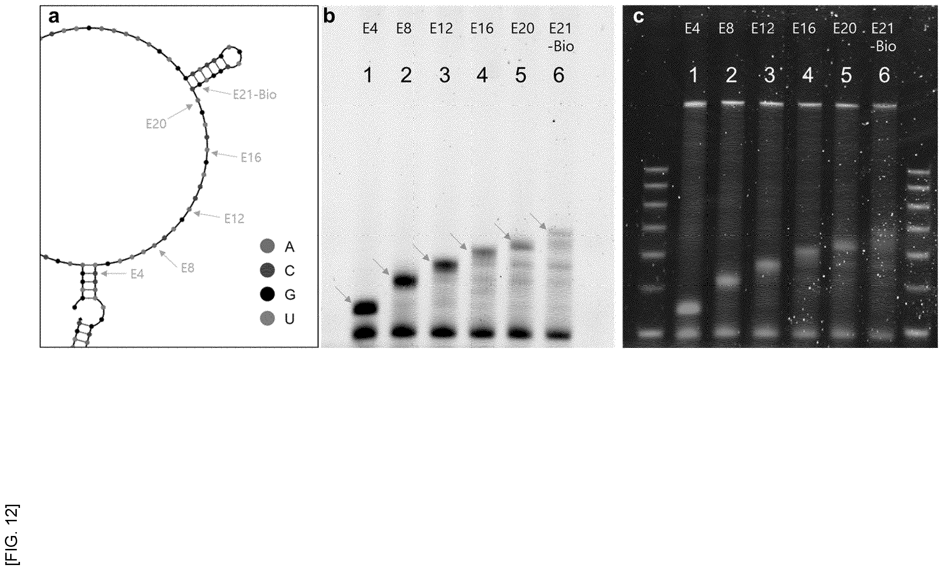

As above, it showed that MOPSS is also possible for pegRNA (FIG. 12).

The same MOPSS was performed, but with longer MOPSS cycles (8 cycles for sgRNA and 20 cycles for pegRNA).

The simulation structure of the pegRNA at the temperature of the MOPSS run (FIG. 12a) and the DNA bands that grew to the target length in the corresponding MOPSS cycle (FIG. 12b) are indicated by gray or blue arrows.

Due to the structural complexity of pegRNA and the longer cycles of MOPSS than sgRNA, fewer end products than sgRNA were observed, but it was shown that the MOSS products were selectively collected by finally proceeding to capture, demonstrating that pegRNA can also be purified via MOPSS (lane 6 of FIG. 12b and FIG. 12c).

While the foregoing has described in detail certain aspects of the present invention, it will be apparent to one of ordinary skill in the art that these specific descriptions are merely preferred examples and are not intended to limit the scope of the invention. Accordingly, the substantial scope of the present invention will be defined by the appended claims.

## Claims

1. A method of purifying a target nucleic acid, comprising:
(a) hybridizing a library containing the target nucleic acid with a primer that binds complementarily to the target nucleic acid;
(b) reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid, wherein, when one of dATP, dCTP, dGTP and dTTP is selected as a blocker-free dNTP that binds complementarily to a predetermined nucleotide of the target nucleic acid, the NTPs other than the selected blocker-free NTP comprise an irreversible blocker,
wherein the elongation reaction comprises alternating reaction cycles of NTPs comprising an irreversible blocker and blocker-free NTPs; and
(c) isolating the target nucleic acid to which the complementary nucleic acid is bound from the reactants of step (b),
wherein the NTPs comprising the irreversible blocker of step (b) are dideoxy nucleoside triphosphates (ddNTPs) or pyrrolidinyl nucleoside triphosphates (prNTPs).

2. A method for analyzing the presence of a target nucleic acid in a sample, comprising:
(a) hybridizing a sample containing the target nucleic acid with a primer that binds complementarily to the target nucleic acid;
(b) reacting the hybridization reactant of step (a) with a preparation comprising (i) a nucleotide triphosphate (NTP) comprising an irreversible blocker and (ii) a blocker-free deoxy NTP selected from the group consisting of blocker-free dATP, dCTP, dGTP, and dTTP that binds complementarily to a predetermined nucleotide of the target nucleic acid, wherein, when one of dATP, dCTP, dGTP and dTTP is selected as a blocker-free NTP that binds complementarily to a predetermined nucleotide of the target nucleic acid, the NTPs comprising an irreversible blocker have other than the selected blocker-free NTP,
wherein the elongation reaction comprises alternating reaction cycles of NTPs comprising an irreversible blocker and blocker-free NTPs; and
(c) isolating the target nucleic acid to which the complementary nucleic acid is bound from the reactants of step (b),
wherein the NTPs comprising the irreversible blocker of step (b) are dideoxy nucleoside triphosphates (ddNTPs) or pyrrolidinyl nucleoside triphosphates (prNTPs).

3. The method according to claim 1 or 2, wherein the reaction in step (b) results in the binding of a nucleic acid complementary to the target nucleic acid and mismatches to the non-target nucleic acid, such that the reaction is stopped by an NTP comprising an irreversible blocker.

4. The method according to claim 1 or 2, wherein step (c) involves attaching a tag to the NTP containing an irreversible blocker and separating the product, in which the reaction has been halted by the NTP containing the irreversible blocker, from the target nucleic acid to which the complementary nucleic acid is bound.

5. The method according to claim 1 or 2, further comprising the step of (d) isolating only the target nucleic acid from the target nucleic acid to which the complementary nucleic acid is bound.

6. The method according to claim 5, wherein step (d) is performed by treating the target nucleic acid to which the complementary nucleic acid is bound with a nuclease.

7. The method according to claim 1 or 2, wherein step (b) comprises an NTP comprising an irreversible blocker at a concentration of 20 to 200 µM.

8. The method according to claim 1 or 2, wherein the preparation of step (b) further comprises an enzyme, a buffer, and a salt.

9. The method according to claim 7, wherein the salt comprises MnCl₂ or MnSO₄.

10. The method according to claim 9, wherein the salt comprises a concentration of 1 mM or more.

11. The method according to any of claims 1 to 10, wherein the target nucleic acid is ribonucleic acid.

## Patentansprüche

1. Verfahren zum Reinigen einer Ziel-Nukleinsäure, das Folgendes umfasst:
(a) das Hybridisieren einer Bibliothek, welche die Ziel-Nukleinsäure enthält, an einen Primer, der komplementär an die Ziel-Nukleinsäure bindet;
(b) das Umsetzen des Hybridisierungs-Reaktanden aus Schritt (a) mit einem Präparat, das (i) ein Nukleotidtriphosphat (NTP), das einen irreversiblen Blocker umfasst, und (ii) ein Blocker-freies Desoxy-NTP, das aus der aus Blocker-freiem dATP, dCTP, dGTP und dTTP bestehenden Gruppe ausgewählt ist und das komplementär an ein vorbestimmtes Nukleotid der Ziel-Nukleinsäure bindet, umfasst, wobei, wenn eines aus dATP, dCTP, dGTP und dTTP als Blocker-freies dNTP ausgewählt wird, das komplementär an ein vorbestimmtes Nukleotid der Ziel-Nukleinsäure bindet, die NTP, die nicht das ausgewählte Blocker-freie NTP sind, einen irreversiblen Blocker umfassen,
wobei die Verlängerungsreaktion das Abwechseln von Reaktionszyklen von NTP, die einen irreversiblen Blocker umfassen, und von Blocker-freien NTP umfasst; und
(c) das Isolieren der Ziel-Nukleinsäure, an welche die komplementäre Nukleinsäure gebunden ist, von den Reaktanden aus Schritt (b);
wobei die NTP, die den irreversiblen Blocker umfassen, aus Schritt (b) Didesoxynukleosidtriphosphate (ddNTP) oder Pyrrolidinylnukleosidtriphosphate (prNTP) sind.

2. Verfahren zum Analysieren der Gegenwart einer Ziel-Nukleinsäure in einer Probe, das Folgendes umfasst:
(a) das Hybridisieren einer Probe, welche die Ziel-Nukleinsäure enthält, an einen Primer, der komplementär an die Ziel-Nukleinsäure bindet;
(b) das Umsetzen des Hybridisierungs-Reaktanden aus Schritt (a) mit einem Präparat, das (i) ein Nukleotidtriphosphat (NTP), das einen irreversiblen Blocker umfasst, und (ii) ein Blocker-freies Desoxy-NTP, das aus der aus Blocker-freiem dATP, dCTP, dGTP und dTTP bestehenden Gruppe ausgewählt ist und das komplementär an ein vorbestimmtes Nukleotid der Ziel-Nukleinsäure bindet, umfasst, wobei, wenn eines aus dATP, dCTP, dGTP und dTTP als Blocker-freies NTP ausgewählt wird, das komplementär an ein vorbestimmtes Nukleotid der Ziel-Nukleinsäure bindet, die NTP, die nicht das ausgewählte Blocker-freie NTP sind, einen irreversiblen Blocker umfassen,
wobei die Verlängerungsreaktion das Abwechseln von Reaktionszyklen von NTP, die einen irreversiblen Blocker umfassen, und von Blocker-freien NTP umfasst; und
(c) das Isolieren der Ziel-Nukleinsäure, an welche die komplementäre Nukleinsäure gebunden ist, von den Reaktanden aus Schritt (b);
wobei die NTP, die den irreversiblen Blocker umfassen, aus Schritt (b) Didesoxynukleosidtriphosphate (ddNTP) oder Pyrrolidinylnukleosidtriphosphate (prNTP) sind.

3. Verfahren nach Anspruch 1 oder 2, wobei die Reaktion in Schritt (b) zur Bindung einer Nukleinsäure führt, die zu der Ziel-Nukleinsäure komplementär ist und eine Fehlanpassung mit der Nicht-Ziel-Nukleinsäure aufweist, so dass die Reaktion durch ein NTP, das einen irreversiblen Blocker umfasst, gestoppt wird.

4. Verfahren nach Anspruch 1 oder 2, wobei Schritt (c) das Anbinden einer Markierung an das NTP, das einen irreversiblen Blocker enthält, und das Abtrennen des Produkts, in dem die Reaktion durch das NTP, das den irreversiblen Blocker enthält, gestoppt wurde, von der Ziel-Nukleinsäure, an welche die komplementäre Nukleinsäure gebunden ist, umfasst.

5. Verfahren nach Anspruch 1 oder 2, das weiters den Schritt (d) des Isolierens nur der Ziel-Nukleinsäure von der Ziel-Nukleinsäure, an welche die komplementäre Nukleinsäure gebunden ist, umfasst.

6. Verfahren nach Anspruch 5, wobei Schritt (d) durch Behandeln der Ziel-Nukleinsäure, an welche die komplementäre Nukleinsäure gebunden ist, mit einer Nuklease durchgeführt wird.

7. Verfahren nach Anspruch 1 oder 2, wobei Schritt (b) ein NTP, das einen irreversiblen Blocker umfasst, in einer Konzentration von 20 bis 200 µM umfasst.

8. Verfahren nach Anspruch 1 oder 2, wobei das Präparat aus Schritt (b) weiters ein Enzym, einen Puffer und ein Salz umfasst.

9. Verfahren nach Anspruch 7, wobei das Salz MnCl₂ oder MnSO₄ umfasst.

10. Verfahren nach Anspruch 9, wobei das Salz eine Konzentration von 1 mM oder mehr aufweist.

11. Verfahren nach einem der Ansprüche 1 bis 10, wobei die Ziel-Nukleinsäure Ribonukleinsäure ist.

## Revendications

1. Procédé de purification d'un acide nucléique cible, comprenant les étapes consistant à :
(a) hybrider une bibliothèque contenant l'acide nucléique cible avec une amorce qui se lie de manière complémentaire à l'acide nucléique cible ;
(b) faire réagir le réactif d'hybridation de l'étape (a) avec une préparation comprenant (i) un nucléotide triphosphate (NTP) comprenant un bloqueur irréversible et (ii) un désoxy NTP sans bloqueur, choisi dans le groupe constitué de dATP, dCTP, dGTP et dTTP sans bloqueur, qui se lie de manière complémentaire à un nucléotide prédéterminé de l'acide nucléique cible, dans lequel, lorsque l'un des dATP, dCTP, dGTP et dTTP est choisi comme dNTP sans bloqueur qui se lie de manière complémentaire à un nucléotide prédéterminé de l'acide nucléique cible, les NTP autres que le NTP sans bloqueur choisi comprennent un bloqueur irréversible,
dans lequel la réaction d'allongement comprend des cycles de réaction alternés de NTP comprenant un bloqueur irréversible et sans bloqueur ; et
(c) isoler l'acide nucléique cible auquel l'acide nucléique complémentaire est lié des réactifs de l'étape (b),
dans lequel les NTP comprenant le bloqueur irréversible de l'étape (b) sont des didésoxy nucléoside triphosphates (ddNTP) ou des pyrrolidinyl nucléoside triphosphates (prNTP).

2. Procédé d'analyse de la présence d'un acide nucléique cible dans un échantillon, comprenant les étapes consistant à :
(a) hybrider un échantillon contenant l'acide nucléique cible avec une amorce qui se lie de manière complémentaire à l'acide nucléique cible ;
(b) faire réagir le réactif d'hybridation de l'étape (a) avec une préparation comprenant (i) un nucléotide triphosphate (NTP) comprenant un bloqueur irréversible et (ii) un désoxy NTP sans bloqueur, choisi dans le groupe constitué de dATP, dCTP, dGTP et dTTP sans bloqueur, qui se lie de manière complémentaire à un nucléotide prédéterminé de l'acide nucléique cible, dans lequel, lorsque l'un des dATP, dCTP, dGTP et dTTP est choisi comme NTP sans bloqueur qui se lie de manière complémentaire à un nucléotide prédéterminé de l'acide nucléique cible, les NTP comprenant un bloqueur irréversible sont autres que le NTP sans bloqueur choisi,
dans lequel la réaction d'allongement comprend des cycles de réaction alternés de NTP comprenant un bloqueur irréversible et sans bloqueur ; et
(c) isoler l'acide nucléique cible auquel l'acide nucléique complémentaire est lié des réactifs de l'étape (b),
dans lequel les NTP comprenant le bloqueur irréversible de l'étape (b) sont des didésoxy nucléoside triphosphates (ddNTP) ou des pyrrolidinyl nucléoside triphosphates (prNTP).

3. Procédé selon la revendication 1 ou 2, dans lequel la réaction à l'étape (b) entraîne la liaison d'un acide nucléique complémentaire de l'acide nucléique cible et des mésappariements à l'acide nucléique non cible, de sorte que la réaction est arrêtée par un NTP comprenant un bloqueur irréversible.

4. Procédé selon la revendication 1 ou 2, dans lequel l'étape (c) consiste à attacher un marqueur au NTP contenant un bloqueur irréversible et à séparer le produit, dans lequel la réaction a été arrêtée par le NTP contenant le bloqueur irréversible, de l'acide nucléique cible auquel l'acide nucléique complémentaire est lié.

5. Procédé selon la revendication 1 ou 2, comprenant en outre l'étape consistant à (d) isoler uniquement l'acide nucléique cible de l'acide nucléique cible auquel l'acide nucléique complémentaire est lié.

6. Procédé selon la revendication 5, dans lequel l'étape (d) est effectuée en traitant l'acide nucléique cible auquel l'acide nucléique complémentaire est lié avec une nucléase.

7. Procédé selon la revendication 1 ou 2, dans lequel l'étape (b) comprend un NTP comprenant un bloqueur irréversible à une concentration de 20 à 200 µM.

8. Procédé selon la revendication 1 ou 2, dans lequel la préparation de l'étape (b) comprend en outre une enzyme, un tampon et un sel.

9. Procédé selon la revendication 7, dans lequel le sel comprend MnCl₂ ou MnSO₄.

10. Procédé selon la revendication 9, dans lequel le sel comprend une concentration de 1 mM ou plus.

11. Procédé selon l'une quelconque des revendications 1 à 10, dans lequel l'acide nucléique cible est de l'acide ribonucléique.
